# FASCICULE DE BREVET EUROPEEN

(11) **EP 3 212 245 B1**
(45) Date de publication et mention de la délivrance du brevet: **11.09.2024**
(21) Numéro de dépôt: 15786941.3
(22) Date de dépôt: 27.10.2015
(51) Int. Cl.: A61L 27/10, A61L 27/54, A61L 27/56, A61K 31/498, A61P 19/00, A61P 35/00, A61P 35/04, C04B 38/06

(54) **COMPOSITION POREUSE CHARGÉE EN PRINCIPE ACTIF**
MIT EINEM WIRKSTOFF GEFÜLLTE PORÖSE ZUSAMMENSETZUNG
POROUS COMPOSITION FILLED WITH AN ACTIVE INGREDIENT

(30) Priorité: 27.10.2014 FR 1460316
(43) Date de publication de la demande: 06.09.2017
(73) Titulaire: I.Ceram, 87280 Limoges (FR)
(72) Inventeur: BERTIN, François, 87042 Limoges cedex (FR); DENES, Eric, 87000 Limoges (FR); FIORENZA, Fabrice, 87042 Limoges cedex (FR); STURTZ, Franck, 87042 Limoges cedex (FR); SETTON, Daniel, 87038 Limoges (FR)
(74) Mandataire: August Debouzy
(86) Numéro de dépôt international: PCT/EP2015/074910
(87) Numéro de publication internationale: WO 2016/066660

(56) Documents cités:
- EP-A1- 0 505 634
- WO-A2-2012/090070
- US-A- 4 192 021
- US-A- 5 626 861
- US-A1- 2011 117 165
- US-A1- 2011 182 965
- US-B1- 6 417 247
- US-B1- 6 989 033
- US-B2- 7 037 304
- DATABASE WPI Section Ch Week 199837, Derwent World Patents Index; Class A96, AN 1998-431426, XP002741194, KIKUTANI T; SHIBUYA T: "Biologically active cement composition for bonding fixation of implant materials, supplementation filling of bone deficit part and artificial bones - consists of specific alumina powder, dimethacrylate group monomer, polymerisation initiator, promoter, retarder and osteogenesis factor"
- IKADA Y ET AL: "Release of antibiotic from composites of hydroxyapatite and poly(lactic acid)", JOURNAL OF CONTROLLED RELEASE, ELSEVIER, AMSTERDAM, NL, vol. 2, 1 November 1985 (1985-11-01), pages 179 - 186, XP025943002, ISSN: 0168-3659, [retrieved on 19851101], DOI: 10.1016/0168-3659(85)90043-4

## Description

### DOMAINE DE L'INVENTION

L'invention concerne une composition pour la libération d'un principe actif, comprenant une matrice poreuse en céramique, chargée avec un vecteur comprenant un principe actif.

### ARRIERE-PLAN TECHNIQUE

Lors d'interventions chirurgicales dans le traitement des cancers de l'os, qu'ils soient primaires ou secondaires, et notamment pour le traitement des métastases osseuses, ou dans le cas d'infection osseuse, le chirurgien est amené à supprimer la partie malade/infectée de l'os. Il est alors parfois nécessaire de la remplacer. Plusieurs techniques existent à cette fin (par exemple amputation, exérèse importante autour de la tumeur, mise en place d'une prothèse et système d'allongement de type Illizaroff), mais elles présentent toutes un problème majeur qui est la survenance d'une infection.

Il existe donc un besoin de mettre au point une prothèse ou implant qui ne présente pas les inconvénients précités.

Le document US 6,989,033 concerne un implant pour créer, recréer ou stabiliser au moins partiellement des corps vertébraux ou des os tubulaires. Dans ledit implant, un corps creux métallique, non métallique ou céramique est revêtu d'un complexe de substances actives ou comprend ledit complexe de substances actives. Ce complexe de substances actives comprend les composants suivants, différents les uns des autres et spécifiquement adaptés à la création d'os : au moins un composant structurel à base de matériau extracellulaire spécifiquement adapté aux cellules de l'os à créer, au moins un composant de recrutement, au moins un composant d'adhésion, et au moins un composant de croissance et/ou de maturation.

Le document US 7,037,304 concerne un bouclier anti-infectieux implantable et un système d'administration de médicaments dans le tissu vasculaire comprennent un biomatériau fibreux poreux relativement non biodégradable qui contrôle et dirige la croissance cellulaire et l'angiogenèse du tissu vasculaire adjacent vers l'implant.

### RESUME DE L'INVENTION

L'invention a pour objet une composition pour la libération d'un principe actif selon la revendication 1.

Selon une variante de ce mode de réalisation, le gel est un gel PLA-PEG-PLA.

Selon une autre variante de ce mode de réalisation, la composition est susceptible d'être obtenue par imprégnation du gel par mise sous pression.

Un tel précurseur de gel peut être un lyophilisat. La composition peut être susceptible d'être obtenue par imprégnation de la matrice par un liquide puis lyophilisation.

Un tel précurseur de gel peut être une solution de polymère dans un solvant organique. La composition peut être susceptible d'être obtenue par imprégnation de la matrice par ladite solution.

Selon un mode de réalisation, le principe actif est choisi parmi les facteurs de croissance, les analgésiques, les antibiotiques et les anticancéreux, ou une combinaison de deux ou plusieurs.

Selon un mode de réalisation, le principe actif est un antibiotique choisi parmi :
- bétalactamines, notamment amoxicilline, oxacilline, cloxacilline, ceftriaxone, cefotaxime, ceftazidime, piperacilline, imipenen, ertapenem, ceftaroline, aztreonam, cefepime, cefazoline ;
- fluoroquinolones, notamment ofloxacine, ciprofloxacine, levofloxacine, oxifloxacine ;
- aminosides, notamment gentamicine, amikacine ;
- glycopeptides, notamment vancomycine, teicoplanine ;
- clindamycine ;
- clofazimine.

Selon un mode de réalisation, le principe actif est un anticancéreux choisi parmi :
- doxorubicine, cisplatin, methotrexate, ifosfamide, cyclophosphamide, vincristine, dactinomycine, etoposide, denosumab.

L'invention a encore pour objet ladite composition pour son utilisation en thérapie, notamment dans le traitement des cancers des os, en particulier des métastases osseuses.

Selon un mode de réalisation, ladite composition est utile pour son utilisation en thérapie comme blocs de comblements osseux, blocs de corporectomie, cages ou cales intersomatiques du rachis cervical ou lombaire, cales cervicales, cales pour calcaneum, cales d'ostéotomie telles que par les cales tibiales d'addition, cales de dérotation telles que tubérosité tibuale antérieure, de rattrapage, de valgisation, blocs de reconstruction et de comblement, broches, cales et pivots de fixation et d'arthrodèse, pastilles de trépan, blocs d'arthrodèse assurant le maintien de l'espace naturel ou interligne intersomatique, implants spéciaux de reconstruction : bloc de comblement de sinus, plancher et plafond d'orbite, bouchons de craniotomie, implants de comblement et plaques de réhabitation maxillofaciale, et toute pièce anatomique sur laquelle il est possible de réinsérer « nerfs » ou tissus « facia lata ».

Cette composition est une prothèse ou un implant.

### DESCRIPTION DE MODES DE REALISATION DE L'INVENTION

L'invention est maintenant décrite plus en détail et de façon non limitative dans la description qui suit.

En l'absence d'indications contraires, les proportions ou pourcentages indiqués sont massiques.

### Matrice poreuse.

On peut utiliser des matrices poreuses susceptibles de recevoir le gel. De telles matrices sont une céramique (décrite plus en détails infra).

### Céramique.

La matrice céramique de l'invention est à base d'alumine Al₂O₃, qui est poreuse pour permettre le chargement du vecteur et du principe actif. Cette céramique alumine est connue en soi mais elle peut être utilisée dopée par certain autre matériaux type Zircone.

La porosité (ouverte et interconnectée) de cette céramique est de 45 à 75%.

La taille des pores est de 200 à 600 µm, de préférence 400 µm.

La porosité/taille des pores est mesurée par porosimétrie mercure. La porosité est définie par la différence entre le volume occupé par les pores sur le volume total, le volume total étant la somme du volume des pores et de l'alumine. La masse d'alumine étant définie par le volume et la masse volumique, en pesant l'échantillon et en connaissant son volume total, on peut déterminer par différence le volume de pores et donc la porosité (ouverte).

La taille de la matrice céramique est variable et peut aller de quelques millimètres à plusieurs centimètres voire dizaines de centimètres ; le volume peut être compris entre 1 et 250 cm³.

La résistance mécanique à la compression est comprise entre 20 et 60 MPa, avantageusement supérieure à 40 MPa.

Il est connu que l'alumine poreuse peut être préparée selon un procédé comprenant les étapes suivantes :
- (A) fourniture d'un matériau porogène (type mousse, par exemple mousse polyuréthane, servant à régler notamment la porosité et la taille des pores) et imprégnation du matériau porogène par une suspension de particules céramiques alumine (barbotine d'alumine) éventuellement en mélange avec divers additifs organiques tels que liants, plastifiants et dispersants ;
- (B) séchage en étuve ;
- (C) traitement thermique à basse température (inférieure à 700°C) pour éliminer la mousse et des constituants organiques de la suspension ; puis
- (D) frittage à une température supérieure à 1500°C.

Ainsi, l'alumine poreuse peut être avantageusement préparée selon le procédé décrit dans la demande de brevet FR2823674.

La matrice céramique de l'invention est particulièrement préparée en mettant en oeuvre le procédé qui y est décrit. Après la mise en oeuvre des deux premières phases telles que décrites ci-dessus (phases A, B), la pièce céramique poreuse est pré-frittée à une température supérieure à 1200°C, ce qui lui confère une cohésion supérieure (phase C'). Le cycle se poursuit par un nouveau trempage de la pièce dans une autre suspension de particules céramiques (phase E). La viscosité de cette suspension concentrée est contrôlée grâce à divers auxiliaires organiques (liants, plastifiants, dispersants), pour être adaptée à une imprégnation homogène de la pièce poreuse préfrittée. Après un nouveau séchage en étuve (phase B') et pyrolyse des auxiliaires organiques de la suspension (phase C), la pièce céramique est enfin frittée à une température supérieure à 1600°C suivant un cycle adapté (phase D').

Ce procédé de sur-imprégnation renforce les propriétés mécaniques de la céramique frittée et multiplie sa résistance par un coefficient 2, notamment la contrainte à la rupture en compression.

Une telle céramique est disponible chez le déposant, sous la référence Ceramil^{®}.

On peut donner la forme souhaitée à la matrice avant ou après chargement du vecteur (et des principes actifs), ou par un usinage.

### Vecteur.

Le vecteur est une composition qui va permettre au principe actif d'être retenu dans les pores de la céramique. Ce vecteur est tout vecteur susceptible d'être chargé dans les pores de la céramique, et peut notamment être sous la forme de gel ou sous la forme d'un liquide qui pourra être lyophilisé. Le vecteur est approprié pour la libération du principe actif selon un profil de libération choisi.

La matrice de céramique peut être chargée avec la poudre comme suit : on introduit la suspension active de façon liquide ou gélatineuse dans la matrice céramique et on procède ensuite à la lyophilisation.

La forme liquide peut prendre la forme lyophilisée après retraitement. Sous cette forme de liquide le vecteur sera éventuellement susceptible d'être réhydraté une fois l'implant placé dans le corps humain, la forme liquide lyophilisée est convertie *in situ* en un principe de relargage par réhydratation. Le vecteur est efficace sous cette forme réhydratée. Sous cette forme, il est possible de combiner les deux variantes, la réhydratation ayant lieu par contact et pression sanguine tandis que la lyophilisation se fait après mélange du vecteur avec le principe actif, et après chargement dans la matrice.

On préférera le mode de réalisation du gel. On pourra utiliser tout gel approprié comme vecteur pour un principe actif, tels que des hydrogels, des gels à base d'acide polylactique (PLA) ou avec un comonomère (PLGA), des gels à base de diblock ou triblock, etc. Des gels appropriés sont décrits dans les publications suivantes : K. Al-Tahami and J. Singh "Smart Polymer Based Delivery Systems for Peptide and Proteins," Recent Patents on Drug Delivery & Formulation, 1: pages: 65-71 Bentham Science Publishers, 2007, US6592899, US6541033, US6350812, WO2012/090070, WO2014001904 et WO2014001905.

Un gel approprié est celui à base d'un dibloc ou d'un tribloc, notamment PLA-PEG-PLA, et/ou celui objet du brevet WO2012/090070.

Le gel peut avoir une viscosité comprise entre 100 et 1400 mPa.s (entre 100 et 1400 Cp).

On peut charger le gel dans la matrice céramique par divers procédés. Le taux d'incorporation final, défini comme étant le rapport entre le volume de gel polymère chargé sur le volume poreux disponible de la matrice céramique est généralement compris entre 50 et 100%, généralement 100%. Ce taux d'incorporation final est défini comme le taux d'incorporation initial (celui résultant de l'étape de chargement du gel) multiplié par le taux de rétention, ce taux de rétention étant le facteur exprimant la capacité du gel à rester dans la matrice céramique.

Un premier procédé comprend l'étape de mise sous pression pour contraindre le gel à pénétrer la matrice céramique. Avec ce procédé, le taux d'incorporation est élevé, mais le taux de rétention déterminé *in vitro* est d'environ 80%. Ainsi, les taux de rétention mesurés à 1h et 24h, dans les conditions expérimentales *in vitro,* varient de 80-95% à 50-65%. Cependant, une fois implantée *in vivo,* les tissus environnant vont empêcher le gel de sortir rapidement de la matrice, ce qui permettra de conserver un taux d'incorporation élevé, typiquement supérieur à 60%.

Un second procédé comprend l'étape de formation in situ du gel dans la matrice céramique. Dans ce second procédé, le polymère est dissous dans un solvant organique, chargé dans la matrice puis mis en contact avec une solution aqueuse (typiquement un tampon).

La cinétique de libération du principe actif contenu dans le gel ou le vecteur contenu dans la matrice est ajustable de façon connue en soi. En effet, les vecteurs ou gels ont des profils ou cinétiques de libération du principe actif qui peuvent être ajustés. On peut générer des profils de libération spécifiques, adaptés à chaque patient.

Par exemple, un profil de libération dans le corps est tel qu'il existe un premier pic de relargage sur le premier jour et ensuite une décroissance sur les 10 à 30 jours qui suivent. On peut encore prévoir un relargage sensiblement continu.

### Principe actif.

De façon générale, tout principe actif a vocation à être utilisable dans l'invention. Par exemple on pourra utiliser des facteurs de croissance, des analgésiques, des antibiotiques et des anticancéreux/antitumoraux, ou une combinaison de deux ou plusieurs.

Les antibiotiques peuvent être choisis parmi :
- bétalactamines, notamment amoxicilline, oxacilline, cloxacilline, ceftriaxone, cefotaxime, ceftazidime, piperacilline, imipenen, ertapenem, ceftaroline, aztreonam, cefepime, cefazoline ;
- fluoroquinolones, notamment ofloxacine, ciprofloxacine, levofloxacine, oxifloxacine ;
- aminosides, notamment gentamicine, amikacine ;
- glycopeptides, notamment vancomycine, teicoplanine ;
- clindamycine ;
- clofazimine.

Les anticancéreux peuvent être choisis dans la liste des anticancéreux connus, comme par exemple :
- ostéosarcomes et sarcomes osseux à cellules fusiformes: doxorubicine, cisplatin, methotrexate, ifosfamide ;
- sarcomes d'Ewing: doxorubicine, cyclophosphamide, ifosfamide, vincristine, dactinomycine, etoposide ;
- tumeurs à cellules géantes: denosumab ;
- sarcomes des tissus mous: doxorubicine.

### Applications.

L'invention trouve à s'appliquer dans un grand nombre d'utilisations notamment en traumatologie pour les reconstructions de fractures, en orthopédie en tant qu'éléments de comblement, ou comme implants (de fusion du rachis, etc.), pour des ostéotomies d'addition et pour les reconstructions (par exemple maxillo-faciale) et notamment pour les interventions dans le cadre de traitement de cancers de l'os, primaires ou secondaires.

L'invention autorise la fabrication de nombreux substituts osseux et implants, qui peuvent être utilisés comme par exemple en tant que cales d'addition ou en comblement osseux sur l'ensemble du squelette.

Par exemple on peut citer comme application celles des blocs de comblements osseux, blocs de corporectomie, cages ou cales intersomatiques du rachis cervical ou lombaire, cales cervicales, cales pour calcaneum, cales d'ostéotomie (par ex. les cales tibiales d'addition), cales de dérotation (par ex. tubérosité tibiale antérieure), de rattrapage, de valgisation, blocs de reconstruction et de comblement, cales et pivots de fixation et d'arthrodèse, pastilles de trépan, blocs d'arthrodèse assurant le maintien de l'espace naturel ou interligne intersomatique, implants spéciaux de reconstruction : bloc de comblement de sinus, plancher et plafond d'orbite, bouchons de craniotomie, implants de comblement osseux quel que soit le lieux d'implantation sur le squelette, et de façon générale toute pièce de forme anatomique sur laquelle il est possible de réinsérer « nerfs » ou tissus « facia lata » quels que soit le lieu d'implantation sur le squelette.

### EXEMPLES

Les exemples suivants illustrent l'invention sans la limiter.

### Exemple 1.

La céramique alumine est une céramique Ceramil^{®}. Des tests sur 4 échantillons indiquent une bonne homogénéité de la porosité des différentes cales (52±2%) qui seront imprégnées de solutions de polymère. La matrice est de forme cylindrique.

Le gel est un gel triblock PLA-PEG-PLA.

Il peut être chargé dans la matrice par mise en pression ou être formé *in situ* dans la matrice.

Selon le premier procédé de chargement, le gel est introduit par mise sous pression dans la matrice céramique.

On a testé ce procédé avec deux hydrogels, respectivement dur et mou. Ces deux gels sont les suivants (le DMSO est le solvant organique tandis que la phase aqueuse est le tampon PBS) :

| Hydrogel | Polymère | DMSO | PBS |
|---|---|---|---|
| Mou | 24 | 16 | 60 |
| Dur | 42 | 18 | 40 |

Les deux gels peuvent être chargés sans problème par mise sous pression.

La clofazimine est un antibiotique extrêmement hydrophobe et coloré. Cette molécule est incorporée dans les hydrogels et y reste fortement associée du fait de sa forte hydrophobicité. La clofazimine peut donc être utilisée en tant que « marqueur » de l'hydrogel.

### On prépare maintenant 5 hydrogels

Les gels ont été formulés à l'aide de la méthode dite « push-pull » qui consiste à mélanger rapidement le contenu de 2 seringues connectées entre-elles - l'une des seringues contenant une solution de polymère triséquencé PLA-PEG-PLA dissous dans un solvant biocompatible et l'autre contenant une phase aqueuse.

Le polymère utilisé pour cette étude a une masse molaire de l'ordre de 14kD, le solvant biocompatible est du diméthyl sulfoxyde (DMSO) et la phase aqueuse un tampon phosphate (PBS).

En modifiant la concentration du polymère dans le solvant contenu dans l'une des seringues et la proportion entre cette solution et la phase aqueuse, il est possible d'obtenir des gels de différentes compositions. En règle générale, plus le gel contient une proportion importante de polymère, plus sa consistance est importante. Le tableau ci-dessous indique la composition des hydrogels utilisés lors de cette étude.

| Hydrogel | Polymère | DMSO | PBS |
|---|---|---|---|
| 1.1 | 24 | 16 | 60 |
| 1.2 | 30 | 20 | 50 |
| 1.3 | 35 | 15 | 50 |
| 1.4 | 42 | 18 | 40 |
| 1.5 | 50 | 20 | 30 |

La clofazimine est dissoute dans la solution de polymère à une concentration de 1% avant de réaliser le mélange (push-pull) du contenu des 2 seringues. Les gels ainsi obtenus sont fortement colorés en rouge-marron.

Les gels de dureté croissante sont incorporés par pression à l'aide d'une presse. Tous les gels ont pénétré dans les matrices cales sans difficulté sauf le gel le plus dur. Avec ce dernier, la pression exercée sur le poussoir a conduit à la rupture du tube en verre contenant la matrice ; le remplacement du tube en verre permet le chargement de ce gel.

On détermine alors le taux d'incorporation du gel dans la cale en céramique qui est défini comme le rapport entre le volume de gel incorporé et le volume des pores. Le taux d'incorporation ne varie pas sensiblement en fonction de la nature du gel.

| Hydrogel | Taux incorporation |
|---|---|
| 1.1 | 82 |
| 1.2 | 82 |
| 1.3 | 82 |
| 1.4 | 85 |
| 1.5 | 86 |

Les matrices en céramique sont placées dans des tubes Falcon de 15 ml contenant 5 ml de PBS. Au bout de 1h, on observe une sortie d'hydrogel à travers les pores externes de la matrice. Les milieux d'incubation à t=1h sont alors remplacés par 5 ml de tampon PBS. Au bout de 24h, une sortie d'hydrogel est également observée. Les matrices sont légèrement grattées à la spatule pour éliminer le gel présent sur leur surface et les milieux d'incubations sont prélevés. Le gel présent dans les milieux d'incubation est récupéré par centrifugation à 3000 g à température ambiante durant 20 min (centrifugeuse Jouan CR422). La clofazimine incluse dans le gel est alors dosée.

| Hydrogel | Taux rétention 1h | Taux rétention 24h |
|---|---|---|
| 1.1 | 85 | 63 |
| 1.2 | 83 | 53 |
| 1.3 | 83 | 52 |
| 1.4 | 94 | 59 |
| 1.5 | 92 | 60 |

Les hydrogels ont tendance à sortir de la matrice durant toute la durée de l'étude (24h). Toutefois, le taux de rétention reste acceptable puisqu'il est d'environ 85 et 55% respectivement à 1h et 24h.

Afin d'obtenir une matrice céramique remplie de gel, une autre méthode consistant à former le gel directement dans la céramique. Les matrices cales doivent dans un premier temps être imprégnées par une solution organique de polymère. L'incubation des matrices dans un milieu aqueux conduit alors à la formation du gel dans la céramique (formation *in situ*).

Ce second procédé a été testé avec 5 solutions de polymère.

Les solutions organiques sont préparées par dissolution d'un copolymère triséquencé PLA-PEG-PLA de masse molaire 36kD dans de la N-méthyl pyrolidone (NMP) à 50°C durant 5h. Les tests sont réalisés avec des solutions de différentes concentrations : 30, 40, 50, 60 et 70% (wt/wt), 2.1 à 2.5. La clofazimine est incorporée dans ces solutions à une concentration finale de 1% (wt/wt).

Afin de faire pénétrer les solutions organiques dans les matrices à l'aide de pression, une pression exercée manuellement sur le poussoir est suffisante. Les solutions à 30 et 40% sont même capables d'imprégner passivement le gel en 5 min environ sans qu'il soit nécessaire d'appliquer une pression. Il n'y a donc aucune difficulté pour incorporer les solutions de polymère dans les matrices. Cependant, les solutions organiques les plus diluées ont tendance à diffuser hors de la céramique une fois cette dernière retirée du dispositif de mise sous pression, la viscosité n'étant pas suffisante pour maintenir la solution de polymère dans la céramique.

| Précurseur | Taux incorporation |
|---|---|
| 2.1 | 57 |
| 2.2 | 59 |
| 3.3 | 68 |
| 3.4 | 72 |
| 4.5 | 82 |

Le taux d'incorporation est compris entre 57% et 82%, le taux le plus faible étant obtenu avec la solution la moins visqueuse, et le taux le plus élevé avec la solution la plus visqueuse. Ceci provient principalement du fait que les solutions faiblement visqueuses diffusent hors de la matrice une fois celle-ci retirée du dispositif de mise sous pression.

On procède ensuite à l'incubation dans du PBS comme ci-dessus. Les résultats sont les suivants.

| Hydrogel | Taux rétention 1h | Taux rétention 24h |
|---|---|---|
| 2.1 | 99 | 99 |
| 2.2 | 100 | 100 |
| 2.3 | 100 | 100 |
| 2.4 | 100 | 100 |
| 2.5 | 100 | 100 |

Avec la solution la moins visqueuse, une quantité équivalente à environ 1% de la dose initiale a été détectée dans le milieu d'incubation. Dans les autres milieux les quantités détectées sont inférieures à 0,2%.

La formation de gel *in situ* dans la céramique conduit à un taux de rétention qui avoisine les 100% sur une durée de 24h.

On peut ainsi envisager de charger la matrice céramique avec la solution organique de polymère comme précurseur de gel, à une viscosité adaptée (élevée et donc une teneur en polymère élevée dans la solution organique) et implanter la matrice chez le patient, les fluides corporels aqueux entourant la matrice conduisant alors à la formation du gel. On dispose ainsi qu'un second levier sur la cinétique de libération du principe actif chez le patient.

## Revendications

1. Composition pour la libération d'un principe actif, comprenant :
- une matrice poreuse en céramique alumine AbOs ;
- un vecteur chargé dans cette matrice; et
- le principe actif dans le vecteur ;
composition dans laquelle :
- la matrice céramique présente une porosité en volume de 45 à 75%,
- la taille des pores est de 200 à 600 µm, de préférence 400µm,
- le vecteur est un gel ou selon une autre variante le gel est formé *in situ* et le vecteur est un précurseur de gel, et
- la résistance mécanique à la compression est comprise entre 20 et 60 MPa, avantageusement supérieure à 40 MPa,
la porosité et la taille des pores étant déterminées par porosimétrie mercure,
la composition étant une prothèse ou un implant, et
la céramique étant obtenue par imprégnation d'une mousse, préfrittage à une température supérieure à 1200°C, sur-imprégnation par une barbotine, et frittage à une température supérieure à 1600°C.

2. Composition selon la revendication 1, dans laquelle le volume de la matrice est compris entre 1 et 250 cm³.

3. Composition selon l'une quelconque des revendications 1 à 2, dans laquelle le vecteur est un gel PLA-PEG-PLA.

4. Composition selon l'une quelconque des revendications 1 à 3, dans laquelle le taux d'incorporation final du vecteur est compris entre 50 et 100%.

5. Composition selon l'une quelconque des revendications 3 à 4, susceptible d'être obtenue par imprégnation du gel par mise sous pression.

6. Composition selon la revendication 1, dans laquelle le précurseur de gel est un lyophilisat, ladite composition étant susceptible d'être obtenue par imprégnation de la matrice par un liquide puis lyophilisation.

7. Composition selon la revendication 1, dans laquelle le précurseur de gel est une solution de polymère dans un solvant organique, ladite composition étant susceptible d'être obtenue par imprégnation de la matrice par ladite solution.

8. Composition selon l'une quelconque des revendications 1 à 7 pour la libération d'un principe actif, consistant en :
- une matrice poreuse ;
- un vecteur chargé dans cette matrice; et
- le principe actif dans le vecteur.

9. Composition selon l'une quelconque des revendications 1 à 8, dans laquelle le principe actif est choisi parmi les facteurs de croissance, les analgésiques, les antibiotiques et les anticancéreux, ou une combinaison de deux ou plusieurs, ledit anticancéreux étant choisi de préférence parmi : doxorubicine, cisplatin, methotrexate, ifosfamide, cyclophosphamide, vincristine, dactinomycine, etoposide, denosumab, et
ledit antibiotique étant choisi de préférence parmi :
- bétalactamines, notamment amoxicilline, oxacilline, cloxacilline, ceftriaxone, cefotaxime, ceftazidime, piperacilline, imipenen, ertapenem, ceftaroline, aztreonam, cefepime, cefazoline ;
- fluoroquinolones, notamment ofloxacine, ciprofloxacine, levofloxacine, oxifloxacine ;
- aminosides, notamment gentamicine, amikacine ;
- glycopeptides, notamment vancomycine, teicoplanine ;
- clindamycine ;
- clofazimine.

10. Composition selon l'une quelconque des revendications 1 à 9, pour son utilisation en thérapie, notamment dans le traitement des cancers des os, en particulier des métastases osseuses.

11. Composition selon l'une quelconque des revendications 1 à 9, pour son utilisation en thérapie comme blocs de comblements osseux, blocs de corporectomie, cages ou cales intersomatiques du rachis cervical ou lombaire, cales cervicales, cales pour calcaneum, cales d'ostéotomie telles que par les cales tibiales d'addition, cales de dérotation telles que tubérosité tibuale antérieure, de rattrapage, de valgisation, blocs de reconstruction et de comblement, broches, cales et pivots de fixation et d'arthrodèse, pastilles de trépan, blocs d'arthrodèse assurant le maintien de l'espace naturel ou interligne intersomatique, implants spéciaux de reconstruction : bloc de comblement de sinus, plancher et plafond d'orbite, bouchons de craniotomie, implants de comblement et plaques de réhabitation maxillofaciale, et toute pièce anatomique sur laquelle il est possible de réinsérer « nerfs » ou tissus « facia lata ».

## Patentansprüche

1. Zusammensetzung für die Freisetzung eines Wirkstoffs, umfassend:
- eine poröse Keramikmatrix aus Aluminiumoxid Al₂O₃;
- einen in diese Matrix geladenen Vektor; und
- der Wirkstoff in den Vektor;
Zusammensetzung, in der :
- die keramische Matrix hat eine Volumenporosität von 45 bis 75 %,
- die Porengröße beträgt 200 bis 600 µm, vorzugsweise 400µm,
- der Vektor ein Gel ist oder alternativ das Gel *in situ* gebildet wird und der Vektor ein Gelvorläufer ist, und
- die mechanische Druckfestigkeit liegt zwischen 20 und 60 MPa, vorzugsweise über 40 MPa,
Porosität und Porengröße, werden durch Quecksilberporosimetrie bestimmt,
die Zusammensetzung ist eine Prothese oder ein Implantat, und die Keramik wird durch Imprägnieren mit einem Schaum, Vorsintern bei einer Temperatur über 1200°C, Überimprägnieren mit einem Schlicker und Sintern bei einer Temperatur über 1600°C hergestellt.

2. Zusammensetzung nach Anspruch 1, wobei das Volumen der Matrix zwischen 1 und 250 cm³ liegt.

3. Zusammensetzung nach einem der Ansprüche 1 bis 2, wobei der Vektor ein PLA-PEG-PLA-Gel ist.

4. Zusammensetzung nach einem der Ansprüche 1 bis 3, wobei die endgültige Inkorporationsrate des Vektors zwischen 50 und 100 % liegt.

5. Zusammensetzung nach einem der Ansprüche 3 bis 4, die durch Imprägnieren des Gels durch Druckbeaufschlagung erhälich ist.

6. Zusammensetzung nach Anspruch 1, wobei der Gelvorläufer ein Lyophilisat ist, wobei die Zusammensetzung durch Imprägnieren der Matrix mit einer Flüssigkeit und anschließendes Gefriertrocknen erhalten werden kann.

7. Zusammensetzung nach Anspruch 1, wobei der Gelvorläufer eine Lösung eines Polymers in einem organischen Lösungsmittel ist, wobei die Zusammensetzung durch Imprägnieren der Matrix mit der Lösung erhalten werden kann.

8. Zusammensetzung nach einem der Ansprüche 1 bis 7 zur Freisetzung eines Wirkstoffs, bestehend aus:
- eine poröse Matrix ;
- ein geladener Vektor in dieser Matrix; und
- der Wirkstoff in den Vektor.

9. Zusammensetzung nach einem der Ansprüche 1 bis 8, wobei der Wirkstoff aus Wachstumsfaktoren, Analgetika, Antibiotika und Antikrebsmitteln oder einer Kombination von zwei oder mehr ausgewählt ist,
wobei das Antikrebsmittel vorzugsweise ausgewählt ist aus: Doxorubicin, Cisplatin, Methotrexat, Ifosfamid, Cyclophosphamid, Vincristin, Dactinomycin, Etoposid, Denosumab, und
Das Antibiotikum wird vorzugsweise ausgewählt aus:
- Betalaktam-Antibiotika, insbesondere Amoxicillin, Oxacillin, Cloxacillin, Ceftriaxon, Cefotaxim, Ceftazidim, Piperacillin, Imipen, Ertapenem, Ceftarolin, Aztreonam, Cefepim, Cefazolin;
- Fluorchinolone, insbesondere Ofloxacin, Ciprofloxacin, Levofloxacin, Oxifloxacin;
- Aminoglykoside, insbesondere Gentamicin, Amikacin ;
- Glykopeptide, insbesondere Vancomycin, Teicoplanin ;
- Clindamycin;
- Clofazimin.

10. Zusammensetzung nach einem der Ansprüche 1 bis 9 zur Verwendung in der Therapie, insbesondere bei der Behandlung von Knochenkrebs, insbesondere von Knochenmetastasen.

11. Zusammensetzung nach einem der Ansprüche 1 bis 9 zur Verwendung in der Therapie als Knochenauffüllungsblöcke, Korporektomieblöcke, interkorporelle Cages oder Keile für die Hals- oder Lendenwirbelsäule, zervikale Keile, Fersenkeile, Osteotomiekeile wie Tibiaadditionskeile, Unterlegscheiben zur Derotation, wie z. B. Tuberositas anterior tibiae, zum Aufholen, zur Valgisierung, Rekonstruktions- und Füllungsblöcke, Stifte, Unterlegscheiben und Zapfen zur Fixierung und Arthrodese, Bohrereinsätze, Arthrodeseblöcke zur Erhaltung des natürlichen Raums oder der Zwischenkörperlinie, spezielle Rekonstruktionsimplantate: Sinusfüllungsblöcke, Orbitaboden- und -deckenblöcke, Kraniotomiepfropfen, Kiefergelenkfüllungsimplantate und - platten sowie alle anatomischen Teile, bei denen eine Wiedereinsetzung von "Nerven" oder "Facia iata"-Gewebe möglich ist.

## Claims

1. Composition for the release of an active principle, comprising:
- a porous ceramic matrix made from aluminum oxide Al₂O_{3;}
- a vector loaded into this matrix; and
- the active principle in the vector;
composition wherein:
- the ceramic matrix presents a porosity by volume of 45 to 75%,
- the size of the pores is between 200 and 600 µm, preferably 400 µm,
- the vector is a gel, or alternatively the gel is formed *in situ* and the vector is a gel precursor, and
- the mechanical resistance to compression is between 20 and 60 MPa, preferably greater than 40 MPa,
the porosity and size of the pores being determined by mercury porosimetry,
the composition being a prosthesis or implant, and
the ceramic is obtained by impregnation of a foam, pre-sintering at a temperature above 1200°C, over-impregnation with a slip, and sintering at a temperature above 1600°C.

2. Composition according to claim 1, wherein the volume of the matrix is between 1 and 250 cm³.

3. Composition according to anyone of claims 1 to 2, wherein the vector is a PLA-PEG-PLA gel.

4. Composition according to anyone of claims 1 to 3, wherein the final incorporation rate of the vector is between 50 and 100%.

5. Composition according to anyone of claims 3 to 4, obtainable by impregnation of the gel via pressurization.

6. Composition according to claim 1, wherein the gel precursor is a lyophilizate, said composition being obtainable by impregnating the matrix with a liquid and then lyophilization.

7. Composition according to claim 1, wherein the gel precursor is a solution of polymer in an organic solvent, said composition being obtainable by impregnating the matrix with said solution.

8. Composition according to anyone of claims 1 to 7 for the release of an active principle, consisting of:
- a porous matrix;
- a vector loaded into this matrix; and
- the active principle in the vector.

9. Composition according to anyone of claims 1 to 8, wherein the active principle is chosen from among growth factors, analgesics, antibiotics and antineoplastics, or a combination of two or more,
said antineoplastic being preferably selected from: doxorubicin, cisplatin, methotrexate, ifosfamide, cyclophosphamide, vincristine, dactinomycin, etoposide, denosumab, and
said antibiotic being preferably selected from:
- betalactamines, in particular amoxicillin, oxacillin, cloxacillin, ceftriaxone, cefotaxime, ceftazidime, piperacillin, imipenen, ertapenem, ceftaroline, aztreonam, cefepime, cefazoline;
- fluoroquinolones, in particular ofloxacine, ciprofloxacin, levofloxacin, oxifloxacin;
- aminoglycosides, especially gentamicin, amikacin;
- glycopeptides, especially vancomycin, teicoplanin;
- clindamycin;
- clofazimine.

10. Composition according to anyone of claims 1 to 9, for use as a therapy, particularly in the treatment of bone cancers, especially bone metastases.

11. Composition according to anyone of claims 1 to 9, for use as a therapy blocks of bone filler, blocks for corpectomy, cervical or lumbar spinal interbody cages or spacers, cervical spacers, spacers for the calcaneus, osteotomy spacers such as tibial expansion spacers, derotation spacers such as anterior tibial tuberosity, rehabilitative spacers, valgisation spacers, reconstruction and filling blocks, pins, fixation and arthrodesis pivots and spacers, trepan caps, arthrodesis blocks to maintain the natural gap or intervertebral spacing, special reconstruction implants: sinus filler blocks, orbit floor and roof filler blocks, craniotomy plugs, filler implants and maxillofacial reconstruction plates, and any anatomical part on which it is possible to reinsert "nerves" or "facia lata" issue.
